# EUROPÄISCHE PATENTSCHRIFT

(11) **EP 0 917 531 B1**
(45) Veröffentlichungstag und Bekanntmachung des Hinweises auf die Patenterteilung: **09.01.2002**
(21) Anmeldenummer: 97931740.1
(22) Anmeldetag: 02.07.1997
(51) Int. Cl.: C07D 295/12, A61K 7/13, D06P 3/08

(54) **PIPERAZIN-DERIVATE UND OXIDATIONSFÄRBEMITTEL**
PIPERAZINE DERIVATIVES AND OXYDATION DYES
DERIVES DE LA PIPERAZINE ET TEINTURES D'OXYDATION

(30) Priorität: 03.07.1996 DE 19626618; 03.07.1996 DE 19626722
(43) Veröffentlichungstag der Anmeldung: 26.05.1999
(73) Patentinhaber: Hans Schwarzkopf GmbH & Co. KG, 22763 Hamburg (DE)
(72) Erfinder: BITTNER, Andreas, Joachim, D-63701 Offenbach (DE); KLEEN, Astrid, D-40627 Düsseldorf (DE)
(74) Vertreter: Foitzik, Joachim Kurt
(86) Internationale Anmeldenummer: EP9703467
(87) Internationale Veröffentlichungsnummer: WO9801434

(56) Entgegenhaltungen:
- EP-A- 0 008 079
- EP-A- 0 256 468
- EP-A- 0 360 644
- WO-A-91/13881

## Beschreibung

Die Erfindung betrifft Piperazin-Derivate sowie Oxidationsfärbemittel mit diesen Verbindungen.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Gute Oxidationsfarbstoff(-vorprodukt)e müssen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme und den Einfluß chemischer Reduktionsmittel, z. B. gegen Dauerwellflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein.

Als Entwicklerkomponenten werden beispielsweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Amino-pyrazolon-derivate sowie 2,4,5,6-Tetraaminopyrimidin und dessen Derivate eingesetzt.

Als Kupplerkomponenten werden in der Regel m-Phenylendiaminderivate, Naphthole, Resorcin und Resorcinderivate, Pyrazolone, m-Aminophenole sowie Pyridin-Derivate verwendet. Bezüglich der einzelnen verwendbaren Farbstoffkomponenten wird ausdrücklich auf die Colipa-Liste, herausgegeben vom Industrieverband Körperpflege und Waschmittel, Frankfurt, Bezug genommen.

In der Regel gelingt es nicht, mit Hilfe einer einzigen Kuppler/Entwicklerkombination zu natürlichen Farbnuancen zu kommen. In der Praxis ist daher meist eine Kombination verschiedener Entwicklerkomponenten und Kupplerkomponenten erforderlich, um eine natürlich wirkende Färbung zu erhalten.

Viele mit den bekannten Kuppler-Entwickler-Kombinationen erhältliche intensiv-blaue Farbtöne enthalten einen deutlichen Rotanteil. Insbesondere bei helleren Nuancen, aber auch für die Erzielung von Naturnuancen, die eine ausreichende Farbtiefe und eine ausreichende Grauabdeckung erreichen sollen, ist dieser Rotanteil nachteilig.

Es besteht daher nach wie vor der Bedarf an Kuppler-Entwickler-Kombinationen, die eine intensive Färbung im klaren Blaubereich und insbesondere einen reinen Schwarzton ergeben, der weder blau- noch rotstichig ist.

Weiterhin steigt mit wachsender Anzahl der eingesetzten Oxidationsfarbstoffvorprodukte auch das Risiko eines uneinheitlichen Färbeergebnisses, eines schlechteren Egalisierungsvermögens sowie weniger guter Echtheitseigenschaften.

Es besteht somit weiterhin ein Bedarf an neuen Oxidationsfarbstoffvorprodukten, die es insbesondere auch gestatten, natürliche Färbungen auf Basis einer geringeren Zahl von eingesetzten Farbstoffvorprodukten zu erhalten.

Es war daher die Aufgabe der vorliegenden Erfindung, neue Verbindungen zu finden, die die an Oxidationsfarbstoffvorprodukte zu stellenden Anforderungen in besonderem Maße erfüllen.

Überraschenderweise wurde nun gefunden, daß die in der vorliegenden Erfindung beschriebenen Verbindungen der allgemeinen Formel (I) aufgrund ihrer besonderen elektronischen Struktur diese Forderungen besonders gut erfüllen. Insbesondere können mit ihnen Färbungen im "reinen Schwarz" erzielt werden. Weiterhin weisen diese Verbindungen überraschenderweise sowohl ausgeprägte Kuppler- als auch ausgeprägte Entwicklereigenschaften auf. Dadurch werden bereits mit einer geringen Anzahl weiterer Oxidationsfarbstoffvorprodukte vom Kuppler- und/oder Entwicklertyp eine Vielzahl von Farbtönen zugänglich, ohne daß die häufig bei gleichzeitiger Verwendung einer größeren Zahl von Oxidationsfarbstoffvorprodukten beobachteten Egalisierungs- und Echtheitsprobleme auftreten.

Ein erster Gegenstand der vorliegenden Erfindung sind daher Piperazin-Derivate der allgemeinen Formel (I), 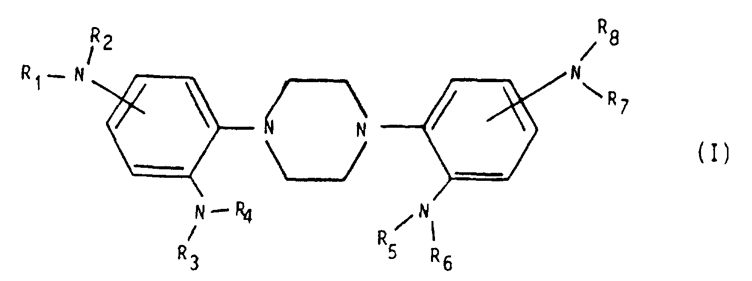 in der R₁ bis R₈ unabhängig voneinander stehen für
- Wasserstoff,
- eine (C₁-C₄)-Alkylgruppe,
- eine Hydroxy-(C₂-C₃)-alkylgruppe,
- eine (C₁-C₄)-Alkoxy-(C₂-C₃)-alkylgruppe,
- eine Amino-(C₂-C₃)-alkylgruppe oder
- eine 2,3-Dihydroxypropylgruppe
mit der Maßgabe,
daß die Gruppen -NR₁R₂ und -NR₇R₈ entweder in den Positionen 4 und 4' oder in den Positionen 5 und 5' stehen,
und deren physiologisch verträgliche Salze mit anorganischen und organischen Säuren.
Diese Verbindungen sind neu, Die Herstellung dieser Verbindungen kann aber nach bekannten Verfahren erfolgen. Hierzu wird ausdrücklich auf die Ausführungen im Beispielteil verwiesen.

Besonders hervorragende Eigenschaften zeigen die erfindungsgemäßen Piperidin-Derivate, bei denen zum einen die Gruppen -NR₁R₂ und -NR₇R₈ und zum anderen die Gruppen -NR₃R₄ und -NR₅R₆ identisch sind.

Bevorzugte Substituenten R₁ bis R₈ sind Wasserstoff, Methyl-, Ethyl-, 2-Hydroxyethyl-, 2-Aminoethyl- sowie 2,3-Dihydroxypropylgruppen.

Die Verbindungen der Formel (I) können sowohl als freie Basen als auch in Form ihrer physiologisch verträglichen Salze mit anorganischen oder organischen Säuren, z.B. der Hydrochloride, der Sulfate und Hydrobromide, vorliegen. Weitere, zur Salzbildung geeignete Säuren sind Phosphorsäure sowie Essigsäure, Propionsäure, Milchsäure und Zitronensäure. Die im weiteren aufgeführten Aussagen zu den Verbindungen gemäß Formel (I) beziehen daher immer diese Salze mit ein.

Die Verbindungen gemäß Formel (I) eignen sich in hervorragender Weise als Oxidationsfarbstoffvorprodukte.

Ein weiterer Gegenstand der vorliegenden Erfindung sind daher Oxidationsfärbemittel zum Färben von Keratinfasern, die Verbindungen gemäß Formel (I) als Oxidationsfarbstoffvorprodukte enthalten.

Unter Keratinfasern sind dabei Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Die erfindungsgemäßen Färbemittel enthalten als zwingende Komponente eine Verbindung der Formel (I) gemäß Anspruch 1 als Oxidationsfarbstoffvorprodukt. Besonders hervorragende Färbeergebnisse wurden bei Verwendung solcher Verbindungen gemäß Formel (I) gefunden, die symmetrisch aufgebaut sind, d.h. bei denen zum einen die Gruppen -NR₁R₂ und -NR₇R₈ und zum anderen die Gruppen -NR₃R₄ und -NR₅R₆ identisch sind. Bezüglich der bevorzugten Substituenten wird auf die bereits oben gemachten Aussagen verwiesen.

Die erfindungsgemäßen Haarfärbemittel enthalten die Verbindungen gemäß Formel (I) bevorzugt in einer Menge von 0,001 bis 10 Gew.-%, vorzugsweise 0,1 bis 5 Gew.-%, jeweils bezogen auf das gesamte Oxidationsfärbemittel. Unter dem "gesamten Oxidationsfärbemittel" oder "gesamten Färbemittel" wird hier und im folgenden das Mittel verstanden, das dem Anwender zur Verfügung gestellt wird. Dieses Mittel kann, je nach Formulierungsform, entweder direkt, oder nach dem Mischen mit Wasser oder z.B. einer wäßrigen Lösung eines Oxidationsmittels auf das Haar aufgebracht werden.

Die Verbindungen gemäß Formel (I) können in den erfindungsgemäßen Oxidationsfärbemitteln sowohl als Entwickler- als auch als Kuppler-Komponenten wirken.

Gemäß einer ersten Ausführungsform enthalten die erfindungsgemäßen Mittel lediglich die Verbindungen der Formel (I) als Oxidationsfarbstoffvorprodukte.

Die Zahl der zugänglichen Farbnuancen wird aber deutlich erhöht, wenn das Mittel neben den Verbindungen gemäß Formel (I) noch mindestens ein weiteres Oxidationsfarbstoffvorprodukt enthält.

Gemäß einer zweiten, bevorzugten Ausführungsform enthalten die erfindungsgemäßen Mittel daher noch mindestens ein weiteres Oxidationsfarbstoffvorprodukt vom Kupplertyp.

Erfindungsgemäß bevorzugte Kupplerkomponenten sind 1-Naphthol, Pyrogallol, 1,5-, 2,7- und 1,7-Dihydroxynaphthalin, o-Aminophenol, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 4-Chlorresorcin, 2-Chlor-6-methyl-3-aminophenol, 2-Methylresorcin, 5-Methylresorcin, 2,5-Dimethylresorcin, 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 4-Amino-2-hydroxytoluol, 2,6-Bis-(2-hydroxyethylamino)-toluol, 2,4-Diaminophenoxyethanol, 2-Amino-4-hydroxyethylamino-anisol.

Erfindungsgemäß besonders bevorzugt sind 1,7-Dihydroxynaphthalin, m-Aminophenol, 2-Methylresorcin, 4-Amino-2-hydroxytoluol, 2-Amino-4-hydroxyethylamino-anisol und 2,4-Diaminophenoxyethanol.

Selbstverständlich umfaßt diese Ausführungsform auch die Verwendung mehrerer zusätzlicher Kupplerkomponenten. Erfindungsgemäß bevorzugte Kupplerkombinationen sind
- Resorcin, m-Phenylendiamin, 4-Chlorresorcin, 2-Amino-4-hydroxyethylaminoanisol
- 2-Methylresorcin, 4-Chlorresorcin, 2-Amino-3-hydroxypyridin
- Resorcin, m-Aminoanilin, 2-Hydroxy-4-aminotoluol
- 3-Methyl-4-aminoanilin, m-Aminoanilin, 2-Hydroxy-4-aminotoluol, 2-Amino-3-hydroxypyridin
- 2-Methylresorcin, m-Aminoanilin, 2-Hydroxy-4-aminotoluol, 2-Amino-3-hydroxypyridin

Gemäß einer zweiten, bevorzugten Ausführungsform enthalten die erfindungsgemäßen Mittel daher, gewünschtenfalls neben einem weiteren Oxidationsfarbstoffvorprodukt vom Kupplertyp, noch mindestens ein weiteres Oxidationsfarbstoffvorprodukt vom Entwicklertyp.

Erfindungsgemäß bevorzugte Entwicklerkomponenten sind p-Phenylendiamin, p-Toluylendiamin, p-Aminophenol, 3-Methyl-1,4-diaminobenzol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5, 4-Amino-3-methylphenol, 2-Methylamino-4-aminophenol, 2,4,5,6-Tetraaminopyrimidin, 2-Hydroxy-4,5,6-triaminopyrimidin, 4-Hydroxy-2,5,6,-triaminopyrimidin, 2,4-Dihydroxy-5,6-Diaminopyrimidin, 2-Dimethylamino-4,5,6-triaminopyrimidin und 2-Hydroxyethylaminomethyl-4-amino-phenol.

Erfindungsgemäß ganz besonders bevorzugt sind p-Toluylendiamin, p-Aminophenol, 1-(2'-Hydroxyethyl)-2.5-diaminobenzol, 4-Amino-3-methylphenol, 2-Methylamino-4-aminophenol und 2,4,5,6-Tetraaminopyrimidin.

Selbstverständlich umfaßt diese Ausführungsform auch die Verwendung mehrerer zusätzlicher Entwicklerkomponenten. Erfindungsgemäß bevorzugte Kupplerkombinationen sind
- p-Toluylendiamin, p-Phenylendiamin
- 3-Methyl-4-aminoanilin, p-Toluylendiamin
- p-Toluylendiamin, 4-Amino-3-methylphenol
- p-Toluylendiamin, 2-Methylamino-4-aminophenol
- 2,4,5,6-Tetraaminopyrimidin, 1-(2'-Hydroxyethyl)-2,5-diaminobenzol
- 2,4,5,6-Tetraaminopyrimidin, p-Toluylendiamin

Üblicherweise werden Entwicklerkomponenten und Kupplerkomponenten in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten bevorzugt in einem Mol-Verhältnis von 1 : 0,5 bis 1 : 2 im Färbemittel enthalten sein können. Die Gesamtmenge an Oxidationsfarbstoffvorprodukten liegt in der Regel bei höchstens 20 Gew.-%, bezogen auf das gesamte Mittel.

Gemäß einer vierten, ebenfalls bevorzugten Ausführungsform enthalten die erfindungsgemäßen Färbemittel, gegebenenfalls neben weiteren Oxidationsfarbstoffvorprodukten, zur weiteren Modifizierung der Farbnuancen zusätzlich direktziehende Farbstoffe, z.B. aus der Gruppe der Nitrophenylendiamine, Nitroaminophenole, Anthrachinone oder Indophenole. Bevorzugte direktziehende Farbstoffe sind die unter den internationalen Bezeichnungen bzw. Handelsnamen HC Yellow 2, HC Yellow 4, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16, Basic Brown 17, Pikraminsäure und Rodol 9 R bekannten Verbindungen sowie 4-Amino-2-nitrodiphenylamin-2'-carbonsäure, 6-Nitro-1,2,3,4-tetrahydrochinoxalin, (N-2,3-Dihydroxypropyl-2-nitro-4-trifluormethyl)amino-benzol und 4-N-Ethyl-1,4-bis(2'-hydroxyethylamino)-2-nitrobenzol-hydrochlorid. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Färbemittel auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die zwingend oder fakultativ enthaltenen Oxidationsfarbstoffvorprodukte oder die fakultativ enthaltenen direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Übliche Konfektionierungsformen für die erfindungsgemäßen Oxidationsfärbemittel sind Mittel auf Basis von Wasser oder nichtwäßrigen Lösungsmitteln sowie Pulver.

Gemäß einer bevorzugten Ausführungsform zur Herstellung der erfindungsgemäßen Färbemittel werden die Oxidationsfarbstoffvorprodukte in einen geeigneten wasserhaltigen Träger eingearbeitet. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind. Dabei werden die erfindungsgemäßen Färbemittel bevorzugt auf einen pH-Wert von 6,5 bis 11,5 insbesondere von 9 bis 10, eingestellt.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Anionische Tenside können dabei ganz besonders bevorzugt sein.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ether-, Amid- und Hydroxylgruppen sowie in der Regel auch Estergruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare und verzweigte Fettsäuren mit 8 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(-CH₂-CH₂O)ₓ-SO₃ H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylen glykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungs produkte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C8-C22-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethyl-ammoniumglycinate, beispielsweise das Kokosacylaminopropyl-dimethyl-ammonium-glycinat, und 2-Alkyl-3-carboxymethyl-3-hydroxyethyl-imidazoline mit jeweils 8 bis 18 C-Atomen in der Alkyl- oder Acylgruppe sowie das Kokosacyl-aminoethyl-hydroxy-ethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C8-C18-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyl-iminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C12-18-Acylsarcosin.

Nichtionische Tenside enthalten als hydrophile Gruppe z. B. eine Polyolgruppe, eine Polyalkylenglykolethergruppe oder eine Kombination aus Polyol- und Polyglykol-ethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C12-C22-Fettsäuremono- und -diester von Anlagerungsprodukten von 1 bis 30 Mol Ethylenoxid an Glycerin,
- C8-C22-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester,
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride; Dialkyldi-methylammoniumchloride und Trialkylmethylammoniumchloride, z. B. Cetyltrimethyl-ammoniumchlorid, Stearyltrimethylammoniumchlorid, Distearyldimethylammonium-chlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil® -Quat 3270 und 3272 (Hersteller: Th. Goldschmidt; diquatemäre Polydimethylsiloxane, Quaternium-80).

Alkylamidoamine, insbesondere Fettsäureamidoamine wie das unter der Bezeichnung Tego Amid® S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Dialkylammoniummethosulfate und Methyl-hydroxyalkyl-dialkoyloxyalkyl-ammonium-methosulfate.

Ein Beispiel für ein als kationisches Tensid einsetzbares quaternäres Zuckerderivat stellt das Handelsprodukt Glucquat® 100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quatemisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quatemierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose, Maleinsäure und Milchsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- Alkalisierungsmittel wie beispielsweise Ammoniak, Monoethanolamin, 2-Amino-2-methylpropanol und 2-Amino-2-methyl-propandiol-1,3
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N2O, Dimethylether, CO2 und Luft,
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Die oxidative Entwicklung der Färbung kann grundsätzlich mit Luftsauerstoff oder einem in dem Mittel enthaltenen oder diesem unmittelbar vor der Anwendung zugefügten Oxidationsmittel erfolgen.

Gemäß einer ersten, bevorzugten Ausführungsform wird ein chemisches Oxidationsmittel eingesetzt. Dies ist besonders in solchen Fällen vorteilhaft, wenn neben der Färbung ein Aufhelleffekt an menschlichem Haar gewünscht ist. Als Oxidationsmittel kommen insbesondere Wasserstoffperoxid oder dessen Anlagerungsprodukte an Harnstoff, Melamin oder Alkaliborat in Frage. Gemäß einer besonders bevorzugten Variante dieser Ausführungsform wird das erfindungsgemäße Färbemittel unmittelbar vor der Anwendung mit der Zubereitung des Oxidationsmittels, insbesondere einer wäßrigen H₂O₂-Lösung, vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann die Zubereitung mit den Oxidationsfarbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert.

Gemäß einer zweiten Ausführungsform erfolgt die Ausfärbung mit Luftsauerstoff. Dabei ist es vorteilhaft, dem erfindungsgemäßen Färbemittel einen Oxidationskatalysator beizugeben. Geeignete Oxidationskatalysatoren sind Metallsalze bzw. Metallkomplexe, wnhei Übergangsmetalle bevorzugt sein können Die Metallsalze bzw. Metallkomplexe sind bevorzugt in Mengen von 0,0001 bis 1 Gew.-% bezogen auf das gesamte Mittel enthalten. Bevorzugt sind dabei Kupfer, Mangan, Kobalt, Selen, Molybdän, Wismut und Ruthenium-Verbindungen. Kupfer(II)-chlorid, -sulfat und -acetat können bevorzugte Oxidationskatalysatoren sein. Als Metallkomplexe können die Komplexe mit Ammoniak, Ethylendiamin, Phenanthrolin, Triphenylphosphin, 1,2-Diphenylphosphinoethan, 1,3-Diphenylphosphinopropan oder Aminosäuren bevorzugt sein. Selbstverständlich ist es auch möglich, in einem Mittel mehrere Oxidationskatalysatoren einzusetzen. Bezüglich der Herstellung geeigneter Katalysatoren wird ausdrücklich auf die entsprechende Offenbahrung in EP 0 709 365 A 1 (Seite 4, Zeilen 19 bis 42) verwiesen, auf die ausdrücklich Bezug genommen wird.

Weiterhin ist es möglich, die Oxidation mit Hilfe von Enzymen durchzuführen. Dabei können die Enzyme sowohl zur Erzeugung von oxidierenden Per-Verbindungen eingesetzt werden, als auch zur Verstärkung der Wirkung einer geringen Mengen vorhandener Oxidationsmittel. Ein Beispiel für ein enzymatisches Verfahren stellt das Vorgehen dar, die Wirkung geringer Mengen (z.B. 1 % und weniger, bezogen auf das gesamte Mittel) Wasserstoffperoxid durch Peroxidasen zu verstärken.

Ein weiterer Gegenstand der Erfindung ist die Verwendung von Piperazin-Derivaten der allgemeinen Formel (I) gemäß Anspruch 1 zur Färbung von keratinischen Fasern.

Die Verbindungen der allgemeinen Formeln (VII), (VIIa), (VII)' und (IX)' sind Zwischenprodukte zur Herstellung der Verbindungen mit der allgemeinen Formel (I). Ihre Strukturen und Herstellungsverfahren sind im Beispielteil ausführlich dargestellt. Es wurde auch gefunden, daß sich diese Verbindungen als direktziehende Farbstoffe allein oder in Kombination mit anderen direktziehenden Farbstoffen zum Färben von Keratinfasem, insbesondere Haaren, eignen. Es ist weiterhin möglich, diese Verbindungen zusammen mit Haarfarbstoffvorprodukten vom Kuppler- und Entwicklertyp und gegebenenfalls noch weiteren direktziehenden Farbstoffen in Färbemitteln für Keratinfasern, insbesondere Haaren. zu verwenden, bei denen die Farbentwicklung durch Oxidationsmittel oder Luft und Katalysatoren erfolgt.

Die nachfolgenden Beispiele sollen den Erfindungsgegenstand näher erläutern.

### Beispiele

### 1. Allgemeine Herstellungsverfahren

### 1.1. N,N-Bis-(2,4-diaminophenyl)-1,n-piperazine

Die erfindungsgemäßen Verbindungen gemäß Formel (I), die Derivate des N,N'-Bis-(2,4-diaminophenyl)-piperazins darstellen, können nach mehreren Verfahren hergestellt werden, die dem Fachmann im Prinzip bekannt sind. Diese Verfahren sind im folgenden in ihrem Grundprinzip dargestellt und können für die speziellen Verbindungen entsprechend dem allgemeinen Fachwissen modifiziert werden.

Gemäß einem ersten Verfahren werden die erfindungsgemäßen N,N-Bis-(2,4-diaminophenyl)-piperazine der allgemeinen Formel (I) hergestellt, indem 2,4-Dinitrohalogenbenzole der allgemeinen Formel (II), worin X = Fluor, Chlor, Brom oder Iod ist, mit Piperazin (III). in alkalischem Reaktionsmedium gegebenenfalls unter Zusatz von Phasentransferkatalysatoren zu N,N -Bis-(2,4-dinitrophenyl)-piperazinen der allgemeinen Formel (IV) umgesetzt werden.

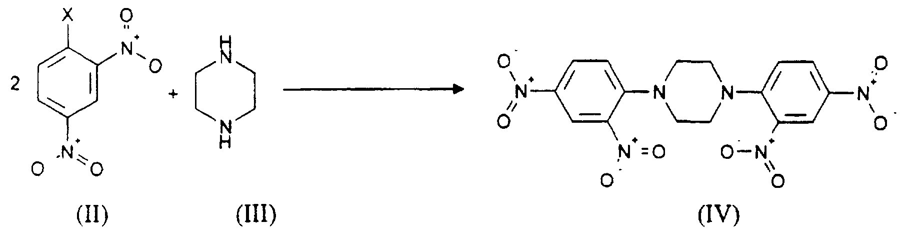

Geeignete Phasentransferkatalysatoren sind beispielsweise Methyl- oder Benzyl-tri(C₆-C₈)alkylammoniumchlorid. Diese Umsetzung kann gegebenenfalls in einem Autoklaven unter Druck erfolgen, um zu einem vollständigen Umsatz zu gelangen. Die Verbindungen der allgemeinen Formel (IV) werden zu den Verbindungen der allgemeinen Formel (V) reduziert, gegebenenfalls zu den erfindungsgemäßen Verbindungen der allgemeinen Formel (I) alkyliert oder oxalkyliert und gegebenenfalls mit anorganischen oder organischen Säuren in deren Salze überführt.

Die Reduktion kann grundsätzlich stufenweise erfolgen, d. h. daß zuerst die beiden orthoständigen Nitrogruppen selektiv reduziert und anschließend alkyliert oder oxalkyliert werden oder daß zuerst die beiden para-ständigen Nitrogruppen einer selektiven Reduktion und anschließend einer Alkylierung oder Hydroxalkylierung unterworfen werden. Die Reduktion der verbleibenden Nitrofunktionen und eventuell die nachfolgende Alkylierung oder Hydroxalkylierung ergibt dann die erfindungsgemäßen Verbindungen der allgemeinen Formel (I).

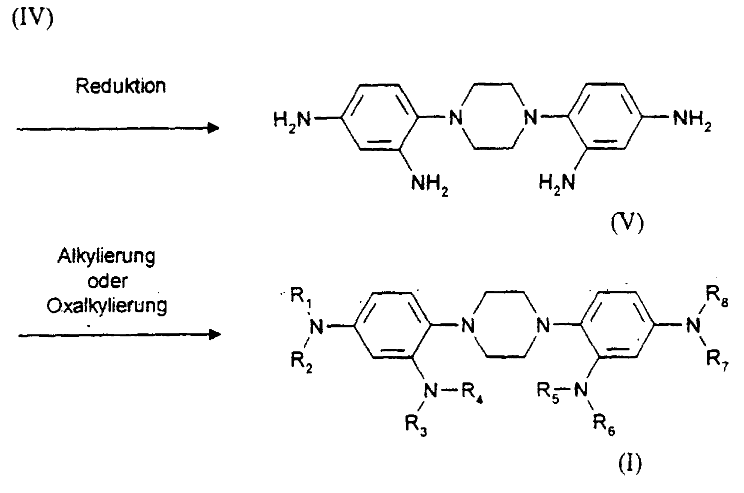

Gemäß einem zweiten Verfahren können die erfindungsgemäßen N,N-Bis-(2,4-diaminophenyl)-piperazine der allgemeinen Formel (I) dadurch erhalten werden, daß substituierte 4-Amino-2-nitrohalogenbenzole der allgemeinen Formel (VI), worin R₁ und R₂ die in Anspruch 1 genannten Bedeutungen haben, mit Piperazin (III) gegebenenfalls unter Zusatz von Phasentransferkatalysatoren zunächst zu Verbindungen der allgemeinen Formel (VII) umgesetzt werden.

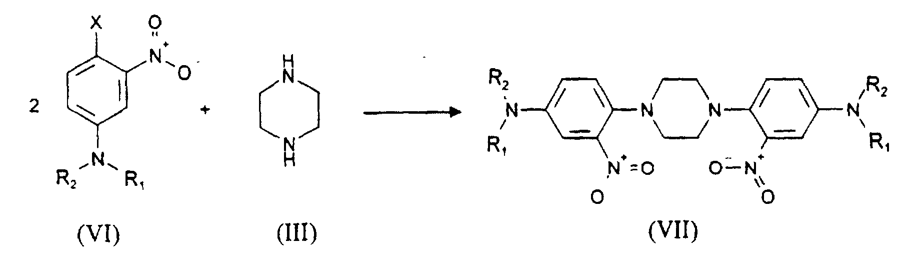

Nach Reduktion und gegebenenfalls weiterer Alkylierung oder Oxalkylierung werden dann die erfindungsgemäßen Verbindungen der allgemeinen Formel (I) erhalten und diese gegebenenfalls mit einer anorganischen oder organischen Säure in ihr Salz überführt.

Nach einem dritten Verfahren können die erfindungsgemäßen N,N-Bis-(2,4-diaminophenyl)-piperazine der allgemeinen Formel (I) dadurch erhalten werden, indem substituierte 2-Amino-4-nitrohalogenbenzole der allgemeinen Formel (VIa), worin R₃ und R₄ die in Anspruch 1 aufgeführte Bedeutung haben, mit Piperazin (III) zunächst zu Verbindungen der allgemeinen Formel (VIIa) umgesetzt werden.

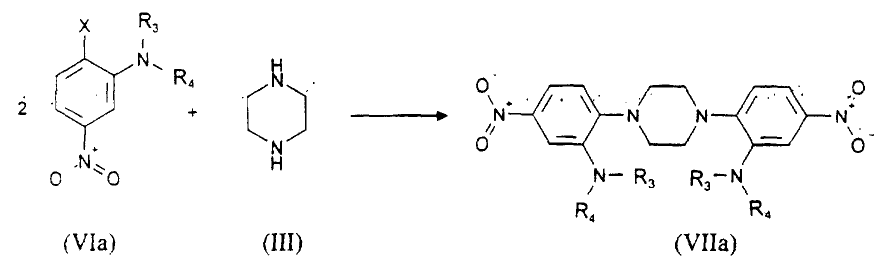

Nach Reduktion und gegebenenfalls weiterer Alkylierung oder Oxalkylierung werden dann die erfindungsgemäßen Verbindungen der allgemeinen Formel (I) erhalten und diese gegebenenfalls mit einer anorganischen oder organischen Säure in ihr Salz überführt.

Die erste Stufe dieser Verfahren besteht prinzipiell im Austausch eines Halogensubstituenten gegen einen Amin-Substituenten am Phenylring. Bei den bekannten Verfahren wird üblicherweise mit einem Aminüberschuß von etwa 40-80% gearbeitet; die Produkte werden in Ausbeuten von ca. 90 % und mit einer Reinheit von 95-96 % erhalten. Überraschenderweise wurde nunmehr gefunden, daß höhere Ausbeuten bei gleicher oder besserer Reinheit und schnellerem Umsatz erzielt werden, wenn der Piperazinüberschuß 30 % und weniger. insbesondere 5 bis 10 Mol-%, bezogen auf die eingesetzte Menge der Verbindung gemäß Formel (II), (VI) oder (VIa) beträgt. Die Umsetzung des Piperazins (Formel (III)) mit Verbindungen der Formel (II), (VI) oder (VIa) erfolgt bevorzugt in Gegenwart von Alkalicarbonaten als säurebindenden Mitteln. Es ist ebenfalls bevorzugt, die Reaktion in einem organischen Lösungsmittel durchzuführen. Diese Reaktion wird bevorzugt unter einem Druck von 1 bis 15 bar, insbesondere von 1 bis 8 bar und ganz besonders bevorzugt von 1 bis 2,5 bar, durchgeführt.

Die Umsetzung der Verbindung der allgemeinen Formel (V), (VII) oder (VIIa) [R₁ und R₂, R₃ und R₄, R₅ und R₆ und/oder R₇ und R₈ = Wasserstoff) kann in Anlehnung an bekannte Verfahren mit Chlorameisensäure-2-chlorethylester oder Chlorameisensäure-3-chlorpropylester erfolgen unter anschließender basischer Behandlung der Chloralkylcarbamate.

Die Herstellung der Verbindungen der allgemeinen Formel (I) kann durch Reduktion der Verbindungen der allgemeinen Formel (IV), (VII) oder (VIIa), gegebenenfalls nach Alkylierung oder Oxalkylierung, mit unedlen Metallen oder durch katalytische Reduktion erfolgen.

Bei der katalytischen Reduktion werden übliche Katalysatoren, z. B. Raney-Nickel, Palladium auf Aktivkohle oder Platin auf Aktivkohle, eingesetzt. Die Reaktionstemperatur liegt zwischen Raumtemperatur und 120 °C, vorzugsweise zwischen 35 und 100 °C; der Druck liegt zwischen Normaldruck und 20 bar, vorzugsweise zwischen 2 und 7 bar. Als Lösungsmittel finden übliche Lösungsmittel wie Wasser, Toluol, Eisessig, niedere Alkohole oder Ether Verwendung. Nach erfolgter Reduktion und Abtrennung des Katalysators kann das Produkt der allgemeinen Formel (I), gegebenenfalls nach Alkylierung oder Oxalkylierung, durch Abziehen des Lösungsmittels unter einem Schutzgas in freier Form isoliert werden. Als Alkylierungsmittel haben sich die bekannten Verbindungen Dimethyl- und Diethylsulfat und als Oxalkylierungsmittel die bekannten Verbindungen Ethylenoxid und Propylenoxid bewährt. Das Produkt nach der allgemeinen Formel (I) wird vorzugsweise unter einem Schutzgas durch Zugabe einer 1,0- bis 1,1-äquivalenten Menge einer Säure in ein Salz überführt, das entweder direkt ausfällt oder nach Abzug des Lösungsmittels erhalten wird.

Als anorganische Säuren sind beispielsweise Salzsäure, Schwefelsäure, Phosphorsäure und als organische Säuren Essigsäure, Propionsäure, Milchsäure oder Citronensäure zur Salzbildung geeignet.

### 1.2. N,N-Bis-(2,5-diaminophenyl)-piperazine

Die erfindungsgemäßen Verbindungen der allgemeinen Formel (I), die Derivate des N,N'-Bis-(2,5-diaminophenyl)-piperazin darstellen, können analog den Derivaten des N,N'-Bis-(2,4-diaminophenyl)-piperazins ebenfalls nach diesen Verfahren hergestellt werden, die dem Fachmann im Prinzip bekannt sind. Diese Verfahren sind im folgenden ebenfalls in ihrem Grundprinzip dargestellt und können für die speziellen Verbindungen entsprechend dem allgemeinen Fachwissen modifiziert werden.

Gemäß einem ersten Verfahren werden die erfindungsgemäßen N,N -Bis-(2,5-diaminophenyl)-piperazine der allgemeinen Formel (I) hergestellt, indem 2,5-Dinitrohalogenbenzole der allgemeinen Formel (II)', worin X = Fluor, Chlor, Brom oder Iod ist, mit Piperazin (III) in alkalischem Reaktionsmedium gegebenenfalls unter Zusatz von Phasentransferkatalysatoren zu N,N'-Bis-(2,5-dinitrophenyl)-piperazinen der allgemeinen Formel (IV)' umgesetzt werden.

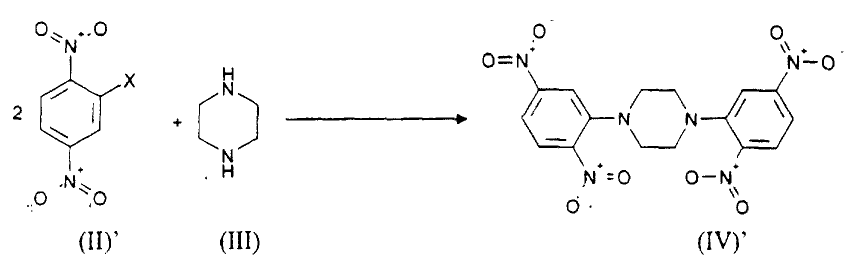

Geeignete Phasentransferkatalysatoren sind beispielsweise Methyl- oder Benzyl-tri(C₆-C₈)-alkylammoniumchlorid. Diese Umsetzung kann gegebenenfalls in einem Autoklaven unter Druck erfolgen, um zu einem vollständigen Umsatz zu gelangen. Die Verbindungen der allgemeinen Formel (IV)' werden zu den Verbindungen der allgemeinen Formel (V)' reduziert, gegebenenfalls zu den erfindungsgemäßen Verbindungen der allgemeinen Formel (I) alkyliert oder oxalkyliert und gegebenenfalls mit anorganischen oder organischen Säuren in deren Salze überführt.

Die Reduktion kann grundsätzlich stufenweise erfolgen, d. h. daß zuerst die beiden orthoständigen Nitrogruppen selektiv reduziert und anschließend alkyliert oder oxalkyliert werden oder daß zuerst die beiden meta-ständigen Nitrogruppen einer selektiven Reduktion und anschließend einer Alkylierung oder Hydroxyalkylierung unterworfen werden. Die Reduktion der verbleibenden Nitrofunktionen und eventuell die nachfolgende Alkylierung oder Hydroxyalkylierung ergibt dann die Verbindungen der allgemeinen Formel (I).

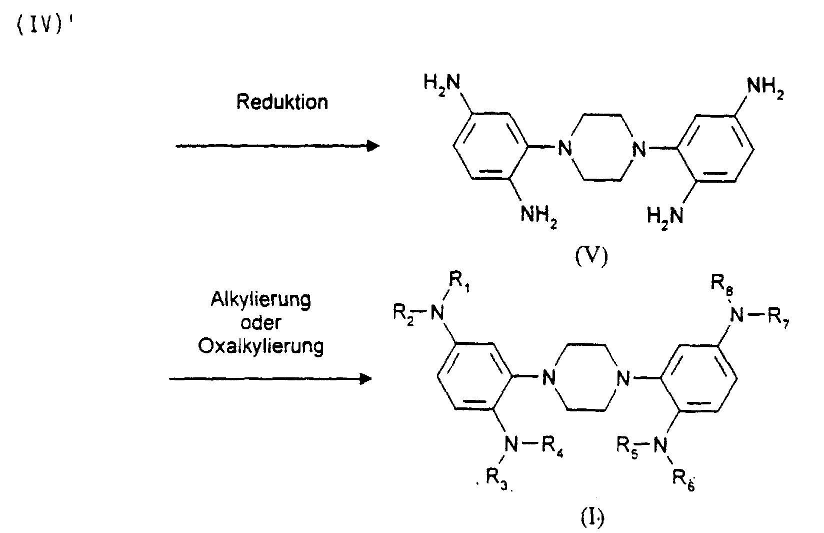

Gemäß einem zweiten Verfahren können die erfindungsgemäßen N,N-Bis-(2,5-diaminophenyl)-piperazine der allgemeinen Formel (I) dadurch erhalten werden, daß substituierte 2-Nitro-5-aminohalogenbenzole der allgemeinen Formel (VI)', worin R₁ und R₂ die vorstehend genannte Bedeutung haben, mit Piperazin (III) zunächst zu Verbindungen der allgemeinen Formel (VII) umgesetzt werden.

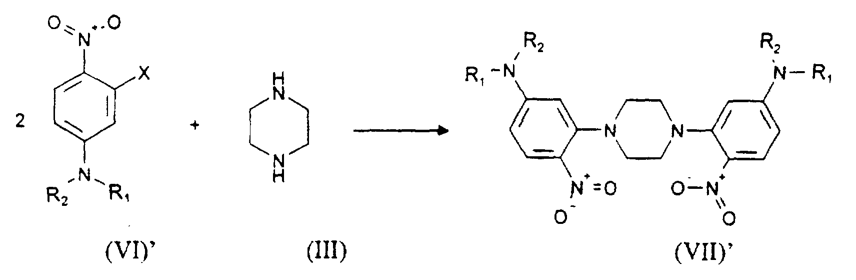

Nach Reduktion und gegebenenfalls weiterer Alkylierung oder Oxalkylierung werden dann die erfindungsgemäßen Verbindungen der allgemeinen Formel (I) erhalten und diese gegebenenfalls mit einer anorganischen oder organischen Säure in deren Salz überführt.

Nach einem dritten Verfahren können die erfindungsgemäßen N,N'-Bis-(2,5-diaminophenyl)-piperazine der allgemeinen Formel (I) dadurch erhalten werden, daß substituierte 2-Nitro-5-aminohalogenbenzole der allgemeinen Formel (VIa)', worin R₁ und R₂ die vorstehend genannte Bedeutung haben, mit Piperazin (III) zunächst zu Verbindungen der allgemeinen Formel (VIII)' umgesetzt werden.

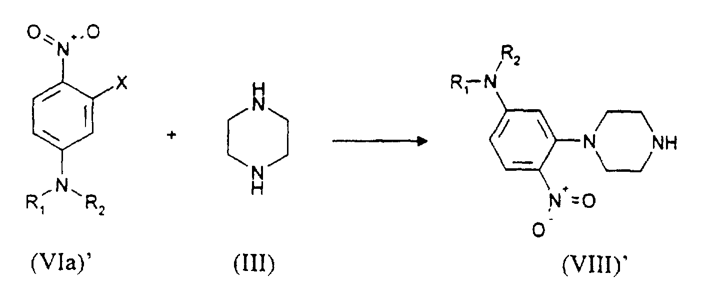

Das Zwischenprodukt der allgemeinen Formel (VIII)' kann nun mit einer Verbindung der allgemeinen Formel (VIb)', die an ihrem Stickstoffatom andere Substituenten R₇ und R₈ mit der oben angegebenen Bedeutung trägt, zu den gemischt substituierten Verbindungen der allgemeinen Formel (IX) umgesetzt werden.

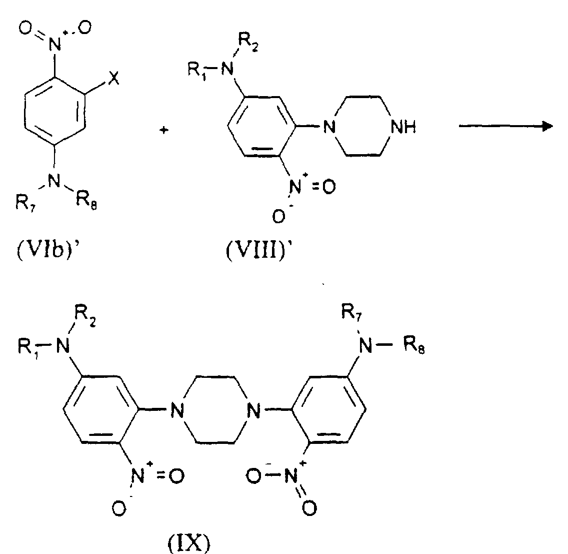

Diese ergeben nach Reduktion und gegebenenfalls weiterer Alkylierung oder Oxalkylierung dann erfindungsgemäße Verbindungen der allgemeinen Formel (I).

Die erste Stufe dieser Verfahren besteht prinzipiell im Austausch eines Halogensubstituenten gegen einen Amin-Substituenten am Phenylring. Bei den bekannten Verfahren wird üblicherweise mit einem Aminüberschuß von etwa 40-80% gearbeitet; die Produkte werden in Ausbeuten von ca. 90 % und mit einer Reinheit von 95-96 % erhalten. Überraschenderweise wurde nunmehr gefunden, daß höhere Ausbeuten bei gleicher oder besserer Reinheit und schnellerem Umsatz erzielt werden, wenn der Aminüberschuß 30 % und weniger, insbesondere 5 bis 10 Mol-%, bezogen auf die eingesetzten Mengen der Verbindung gemäß Formel (II)', (VI)', (VIa)' oder (VIb)', beträgt. Die Umsetzung des Piperazins (Formel (III)) mit Verbindungen der Formel (II)', (VI)', (VIa)' oder (VIb)' erfolgt bevorzugt in Gegenwart von Alkalicarbonaten als säurebindenden Mitteln. Es ist ebenfalls bevorzugt, die Reaktion in einem organischen Lösungsmittel durchzuführen. Diese Reaktion wird bevorzugt unter einem Druck von 1 bis 15 bar, insbesondere von 1 bis 8 bar und ganz besonders bevorzugt von 1 bis 2,5 bar, durchgeführt.
Die Verbindungen der allgemeinen Formel (VII)' oder (IX) mit Substituenten an den Aminogruppen an den Phenylringen sind erhältlich, indem Verbindungen der allgemeinen Formel (VII)' oder (IX), für die R₁ und R₂, oder R₇ und R₈ = Wasserstoff ist, alkyliert oder oxalkyliert werden; dies gelingt, indem man diese Verbindungen in einem inerten Lösungsmittel mit Dialkylsulfat, Alkylhalogenid oder Alkylenoxiden umsetzt oder durch die Umlagerung von daraus hergestellten Carbamaten nach bekannten Verfahren und anschliessende Behandlung mit den vorgenannten Alkylierungsmitteln.

Die Umsetzung der Verbindung der allgemeinen Formel (VII)' oder (IX) [R₁ und R₂ und R₇ und R₈ = Wasserstoff] kann in Anlehnung an bekannte Verfahren mit Chlorameisensäure-2-chlorethylester oder Chlorarneisensäure-3-chlorpropylester erfolgen unter anschließender basischer Behandlung der Chloralkylcarbamate.

Die Herstellung der Verbindungen der allgemeinen Formel (I) kann durch Reduktion der Verbindungen der allgemeinen Formel (IV)', (VII)' oder (IX), gegebenenfalls nach Alkylierung oder Oxalkylierung, mit unedlen Metallen oder durch katalytische Reduktion erfolgen.

Bei der katalytischen Reduktion werden übliche Katalysatoren, z. B. Raney-Nickel, Palladium auf Aktivkohle oder Platin auf Aktivkohle, eingesetzt. Die Reaktionstemperatur liegt zwischen Raumtemperatur und 120 °C, vorzugsweise zwischen 35 und 100 °C, der Druck liegt zwischen Normaldruck und 20 bar, vorzugsweise zwischen 2 und 7 bar. Als Lösungsmittel finden übliche Lösungsmittel wie Wasser, Toluol, Eisessig, niedere Alkohole oder Ether Verwendung. Nach erfolgter Reduktion und Abtrennung des Katalysators kann das Produkt der allgemeinen Formel (I), gegebenenfalls nach Alkylierung oder Oxalkylierung, durch Abziehen des Lösungsmittels unter einem Schutzgas in freier Form isoliert werden. Als Alkylierungsmittel haben sich die bekannten Verbindungen Dimethyl- und Diethylsulfat und als Oxalkylierungsmittel die bekannten Verbindungen Ethylenoxid und Propylenoxid bewährt. Das Produkt nach der allgemeinen Formel (I) wird vorzugsweise unter einem Schutzgas durch Zugabe einer 1,0- bis 1,1-äquivalenten Menge einer Säure in ein Salz überführt, das entweder direkt ausfällt oder nach Abzug des Lösungsmittels erhalten wird.

### 1.3. Herstellung spezieller Verbindungen gemäß Formel (I)

Die hergestellten Verbindungen wurden durch IR- bzw. IR- (KBr-Preßling) und/oder ¹H-NMR-Spektren (in D₆-DMSO) charakterisiert. Soweit die Spektren hier angegeben werden, sind bei den IR-Spektren nur die sehr starken und starken Banden aufgeführt. Bei den Angaben zu den ¹H-NMR-Spektren bedeuten s Singulett, d Dublett, dd Dublett vom Dublett, t Triplett, q Quartett, qi Quintett, m Multiplett, ³J bzw. ⁴J die Kopplungen über drei bzw. vier Bindungen, sowie H³, H⁴, H⁵ und H⁶ die Wasserstoffatome in Position 3, 4, 5 bzw. 6 der Benzolringe.

### 1.3.1. Darstellung von N,N'-Bis-(2,4-diaminophenyl)-piperazin-sulfat

### Stufe a) N,N'-Bis-(2,4-dinitrophenyl)-piperazin

In 55 ml 1,2-Dimethoxyethan wurden 20,3 g (0,1 Mol) 2,4-Dinitrochlorbenzol und 4,3 g (0.05 Mol) Piperazin vorgelegt. Anschließend trug man unter Rühren 4 g (0,1 Mol) Natriumhydroxid (Prills) ein, wobei die Temperatur auf ca. 40 °C anstieg. Dann wurde die Mischung 2 Stunden unter Rückfluß erhitzt, bis die Umsetzung vollständig war. Dann wurden 100ml Wasser zugesetzt und das Produkt ausgerührt. Das ausgefallene Produkt wurde abgesaugt, zweimal mit ca. 100 ml Wasser gewaschen und bei 40 °C im Vakuum getrocknet.
- Ausbeute:: 19,6 g (93,7 % d. Th.)
- IR:: 3114 cm⁻¹ (ν CH_{Ar}), 2926, 2873 cm⁻¹ (ν CH), 1602 cm⁻¹ (ν C=C), 1531, 1510 cm⁻¹ (νₐₛ NO₂), 1327 cm⁻¹ (νₛ NO₂).
- ¹H-NMR:: 8,67 ppm (2 H³, d, ⁴J_{H,H} = 2,68 Hz); 8,34 ppm (2 H⁵, dd, ³J_{H,H} = 9,48 Hz, ⁴J_{H,H} = 2,75 Hz); 7,40 ppm (2 H⁶, d, ³J_{H,H} = 9,50 Hz); 3,64 ppm (8 H, s, NCH₂).

### Stufe b) N,N'-Bis-(2,4-diaminophenyl)-piperazm-Sulfat

In einem 0,3 l-Autoklaven wurden 150 ml Methanol vorgelegt, 16,7 g (0,2 Mol) N,N'-Bis-(2,4-dinitrophenyl)-piperazin gelöst und 2 g Palladium auf Aktivkohle 10 % (Degussa) zugegeben. Nach Verschließen und Inertisieren mit Stickstoff wurde bei einem Druck von 3 bar und einer Temperatur von 35 - 40 °C hydriert, bis kein Wasserstoff mehr aufgenommen wurde. Die Mischung gab man auf einen Druckfilter, löste den Rückstand in 100 ml N-Methylpyrrolidon bei 100 °C auf, filtrierte den Katalysator mit einem Druckfilter ab und tropfte nach Abkühlen des Filtrates auf 5 - 10 °C 9,8 g (0.1 Mol) 80 %ige Schwefelsäure zu. Die gallertige Fällung wurde mit 200 ml Methanol verrührt. Das kristallisierte Produkt wurde abgesaugt, mit Methanol gewaschen und getrocknet.
- Ausbeute:: 16.8 g (84,9 % d. Th.)
- Schmelzpunkt:: > 200 °C

### 1.3.2. Darstellung von N,N'-Bis-{2-amino-4-[di-(2-hydroxyethyl)-amino]phenyl}-piperazin-sulfat

### Stufe a) N,N'-Bis-{2-nitro-4-[di-(2-hydroxyethyl)-amino]phenyl}-piperazin

In einem 0,3 l-Autoklaven wurden 100 ml 1,2-Dimethoxyethan, 24,4 g (0,1 Mol) 4-Fluor-3-nitro-N,N-di-(2-hydroxyethyl)-anilin, 8,6 g (0,1 Mol) Piperazin, 5,3 g (0,05 Mol) Kaliumcarbonat und 0,25 g Methyl-tri(C₆-C₈)alkylammoniumchlorid (70 %ig in *iso*-Propanol) vorgelegt. Diese Mischung wurde 8 Stunden auf 125 °C erhitzt. Nach Abkühlen auf 40 °C wurden die ungelösten Salze abfiltriert und die Mutterlauge mit 100 ml Wasser versetzt. Das ausgefallene Produkt wurde abgesaugt und getrocknet.
- Ausbeute:: 53,5 g (99 % d. Th.)

### Stufe b) N,N'-Bis-{2-amino-4-[di-(2-hydroxyethyl)-amino]phenyl}-piperazin-sulfat

Die Umsetzung in der Stufe b) erfolgt analog zu Beispiel 1.3.1. Stufe b) durch katalytische Reduktion des in Stufe a) erhaltenen Produktes.

### 1.3.3. Darstellung von N,N'-Bis-(2,5-diaminophenyl)-piperazin-sulfat

### Stufe a) N,N'-Bis-(2,5-dinitrophenyl)-piperazin

In 55 ml 1,2-Dimethoxyethan wurden 20,3 g (0,1 Mol) 2,5-Dinitrochlorbenzol und 4,3 g (0,05 Mol) Piperazin vorgelegt. Anschließend trug man unter Rühren 4 g (0,1 Mol) Natriumhydroxid (Prills) ein, wobei die Temperatur auf ca. 40 °C anstieg. Dann erhitzte man die Mischung 2 Stunden unter Rückfluß, bis die Umsetzung vollständig war. Man setzte dann 100 ml Wasser zu und rührte das Produkt aus. Das ausgefallene Produkt wurde abgesaugt, zweimal mit ca. 100 ml Wasser gewaschen und bei 40 °C im Vakuum getrocknet.
- Ausbeute:: 19,6g(93,7% d. Th.)
- IR:: 3114 cm⁻¹ (ν CH_{Ar}), 2926, 2873 cm⁻¹ (ν CH), 1602 cm⁻¹ (ν C=C), 1531, 1510 cm⁻¹ (νₐₛ NO₂), 1327 cm⁻¹ (νₛ NO₂).
- ¹H-NMR:: 8,67 ppm (2 H³, d, ⁴J_{H,H} = 2,68 Hz); 8,34 ppm (2 H⁴, dd, ³J_{H,H} = 9,48 Hz, ⁴J_{H,H} = 2,75 Hz); 7,40 ppm (2 H⁶, d, ³J_{H,H} = 9,50 Hz); 3,64 ppm (8 H, s, NCH₂).

### Stufe b) N,N'-Bis-(2,5-diaminophenyl)-piperazin-Sulfat

In einem 0,3 l-Autoklaven wurden 150 ml Methanol vorgelegt, 16,7 g (0,2 Mol) N,N'-Bis-(2,5-dinitrophenyl)-piperazin gelöst und 2 g Palladium auf Aktivkohle 10 % (Degussa) zugegeben. Nach Verschließen und Inertisieren mit Stickstoff wurde bei einem Druck von 3 bar und einer Temperatur von 35 - 40 °C hydriert, bis kein Wasserstoff mehr aufgenommen wurde. Die Mischung gab man auf einen Druckfilter, löste den Rückstand in 100 ml N-Methylpyrrolidon bei 100 °C auf, filtrierte den Katalysator mit einem Druckfilter ab und tropfte nach Abkühlen des Filtrates auf 5 - 10 °C 9,8 g (0,1 Mol) 80 %ige Schwefelsäure zu. Die gallertige Fällung wurde mit 200 ml Methanol verrührt. Das kristallisierte Produkt wurde abgesaugt, mit Methanol gewaschen und getrocknet.
- Ausbeute:: 16,8 g (84,9 % d. Th.)
- Schmelzpunkt:: > 200 °C

### 1.3.4. Darstellung von N,N'-Bis-(2-amino-5-dimethylaminophenyl)piperazin-sulfat

### Stufe a) N,N'-Bis-(2-nitro-5-dimethylaminophenyl)-piperazin

In einem 0,3 l-Autoklaven wurden 100 ml 1,2-Dimethoxyethan, 20,1 g (0,1 Mol) 2-Nitro-5-N,N-dimethylaminochlorbenzol, 8,6 g (0,1 Mol) Piperazin, 5,3 g (0,05 Mol) Kaliumcarbonat und 0,25 g Methyl-tri(C₆-C₈)alkylammoniumchlorid (70 %ig in iso-Propanol) vorgelegt. Diese Mischung wurde nun 8 Stunden auf 125 °C erhitzt. Nach Abkühlen auf 40 °C wurden die ungelösten Salze abfiltriert und die Mutterlauge mit 100 ml Wasser versetzt. Das ausgefallene Produkt wurde abgesaugt und getrocknet.
- Ausbeute:: 41,0 g (99 % d. Th.)

### Stufe b) N,N'-Bis-(2-amino-5-dimethylaminophenyl)-piperazin-sulfat

Die Umsetzung in der Stufe b) erfolgt analog zu Beispiel 1.3.3. Stufe b) durch katalytische Reduktion des in Stufe a) erhaltenen Produktes.
- Ausbeute:: 20,5 g (45,3 % d. Th.)

### 2. Ausfärbungen

### 2.1. Färbemittel:

- auf Cremebasis C1

| | |
|---|---|
| Natriumlaurylsulfat (70%ig) | 2,5 g |
| Ölsäure | 1,0 g |
| Natriumsulfit, wasserfrei | 0,6 g |
| Cetyl-Stearylalkohol | 12,0 g |
| Myristylalkohol | 6,0 g |
| Propylenglykol | 1,0 g |
| Ammoniak, 25% | 10,0 g |
| Oxidationsfarbstoffvorprodukte | x,xg |
| Wasser | ad 100 g |

- auf Gelbasis G1

| | |
|---|---|
| Ölsäure | 12,0 g |
| Isopropanol | 12,0 g |
| Nonoxynol-4 | 5,0 g |
| Ammoniak, 25 % | 10,0 g |
| Natriumsulfit, wasserfrei | 0,5 g |
| Oxidationsfarbstoffvorprodukte | x,xg |
| Wasser | ad 100 g |

### 2.2. Oxidationsfarbstoffvorprodukte

- gemäß Formel (I)
   (I-1) N,N'-Bis-(2,4-diaminophenyl)-piperazin-sulfat
   (I-2) N,N'-Bis-(2,5-diaminophenyl)-piperazin-sulfat
- Entwickler-Komponenten
   (E-1) p-Aminophenol
   (E-2) 2-(2'-Hydroxyethyl)-p-phenylendiamin-sulfat
   (E-3) p-Phenylendiamin-dihydrochlorid
   (E-4) 2,5-Diaminotoluol-sulfat
   (E-5) 4-Amino-m-cresol
- Kuppler-Komponenten
   (K-1) Resorcin
   (K-2) m-Aminophenol
   (K-3) 4-Amino-2-hydroxytoluol
   (K-4) 2-Amino-4-hydroxyethylamino-anisol-sulfat

### 2.3. Verfahren

50 g des Färbemittels wurden kurz vor dem Gebrauch mit 50 g H₂O₂-Lösung (6%ig in Wasser) gemischt und mittels eines Pinsels auf 100%ig ergrautes Haar (4 g Färbemittel pro g Haar) aufgetragen. Nach einer Einwirkzeit von 30 Minuten bei Raumtemperatur wurde das Färbemittel auf Cremebasis abgespült und das Haar getrocknet. Im Falle der Gel-Basis wurde das Haar nach dem Ausspülen der Farbmasse nachshampooniert und dann erneut gespült und getrocknet.

### 2.4. Ergebnisse

Die Ergebnisse der Ausfärbungen sind in der folgenden Tabelle zusammengestellt:

| Basis | Oxidationsfarbstoffvorprodukte | Farbton |
|---|---|---|
| C1 | 2,47 g I-1 + 1,09 g E-1 | gleichmäßig hellbraunrot |
| C1 | 2,47 g I-1 + 1,52 g E-2 | gleichmäßig silbern |
| C1 | 2,47 g I-1 + 1,08 g E-3 | gleichmäßig schwarz |
| C1 | 2,47 g I-1 + 1,22 g E-4 | gleichmäßig braunsilbern |
| G1 | 2,47 g I-1 + 1,23 g E-5 | hellgoldblond mit rötlichem Reflex |
| C1 | 2,47 g I-1 + 1,10 g K-1 | gleichmäßig olivorange |
| C1 | 2,47 g I-1 + 1,09 g K-2 | gleichmäßig grünblau |
| C1 | 2,47 g I-1 + 1,23 g K-3 | gleichmäßig azurblau |
| C1 | 2,47 g I-1 + 2,80 g K-4 | gleichmäßig marineblau |
| C1 | 2,47 g I-2 + 1,10 g K-1 | gleichmäßig orange mit grünem Reflex |
| C1 | 2,47 g I-2 + 1,09 g K-2 | gleichmäßig blau mit grünem Reflex |
| C1 | 2,47 g I-2 + 1,23 g K-3 | gleichmäßig azurblau |
| C1 | 2,47 g I-2 + 2,80 g K-4 | gleichmäßig marineblau |
| C1 | 2,47 g I-2 + 1,09 g E-1 | gleichmäßig rotbraun |
| C1 | 2,47 g I-2 + 1,52 g E-2 | gleichmäßig silbergrau. |
| C1 | 2,47 g I-2 + 1,08 g E-3 | gleichmäßig blauschwarz |
| C1 | 2,47 g I-2 + 1,22 g E-4 | gleichmäßig braun |
| G1 | 2,47 g I-2 + 1,23 g E-5 | hellgoldblond mit rötlichem Reflex |

## Patentansprüche

1. Piperazin-Derivate der allgemeinen Formel (I), 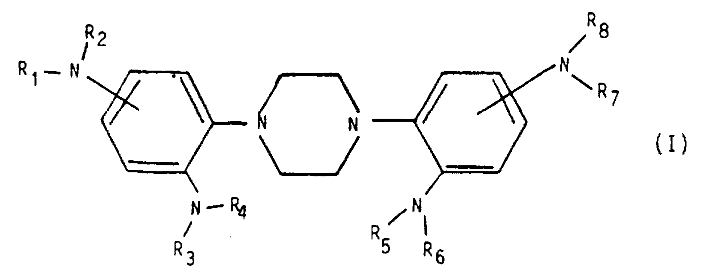 in der R₁ bis R₈ unabhängig voneinander stehen für
- Wasserstoff,
- eine (C₁-C₄)-Alkylgruppe,
- eine Hydroxy-(C₂-C₃)-alkylgruppe,
- eine (C₁-C₄)-Alkoxy-(C₂-C₃)-alkylgruppe,
- eine Amino-(C₂-C₃)-alkylgruppe oder
- eine 2,3-Dihydroxypropylgruppe
mit der Maßgabe,
daß die Gruppen -NR₁R₂ und -NR₇R₈ entweder in den Positionen 4 und 4' oder in den Positionen 5 und 5' stehen,
und deren physiologisch verträgliche Salze mit anorganischen und organischen Säuren.

2. Piperazin-Derivate der allgemeinen Formel (I) nach Anspruch 1, **dadurch gekennzeichnet, daß** die Gruppen -NR₁R₂ und -NR₇R₈ sowie -NR₃R₄ und -NR₅R₆ jeweils identisch sind.

3. Oxidationsfärbemittel zum Färben von Keratinfasern, **dadurch gekennzeichnet, daß** als Oxidationsfarbstoffvorprodukt eine Verbindung gemäß Anspruch 1 oder 2 enthalten ist.

4. Oxidationsfärbemittel gemäß Anspruch 3, **dadurch gekennzeichnet, daß** die Verbindung gemäß Formel (I) in Mengen von 0,001 bis 10 Gew.-%, insbesondere von 0,1 bis 5 Gew.%, bezogen auf das gesamte Mittel, enthalten ist.

5. Oxidationsfärbemittel nach Anspruch 3 oder 4, **dadurch gekennzeichnet, daß** es weiterhin ein Oxidationsfarbstoffvorprodukt vom Kupplertyp enthält.

6. Oxidationsfärbemittel nach einem der Ansprüche 3 bis 5, **dadurch gekennzeichnet, daß** es weiterhin ein Oxidationsfarbstoffvorprodukt vom Entwicklertyp enthält.

7. Oxidationsfärbemittel nach einem der Ansprüche 3 bis 6, **dadurch gekennzeichnet, daß** es weiterhin einen direktziehenden Farbstoff enthält.

8. Oxidationsfärbemittel nach einem der Ansprüche 3 bis 7, **dadurch gekennzeichnet, daß** es weiterhin ein Metallsalz oder einen Metallkomplex enthält.

9. Oxidationsfärbemittel nach Anspruch 8, **dadurch gekennzeichnet, daß** das Metall ausgewählt ist aus Kupfer, Mangan, Kobalt, Selen, Molybdän, Wismut und Ruthenium.

10. Oxidationsfärbemittel nach Anspruch 8 oder 9, **dadurch gekennzeichnet, daß** das Metallsalz oder der Metallkomplex in Mengen von 0,0001 bis 1 Gew.-%, bezogen auf das gesamte Mittel, enthalten ist.

11. Verwendung von Piperazin-Derivaten der allgemeinen Formel (I) gemäß Anspruch 1 zur Färbung von keratinischen Fasern.

## Claims

1. Piperazine derivatives corresponding to general formula (I): 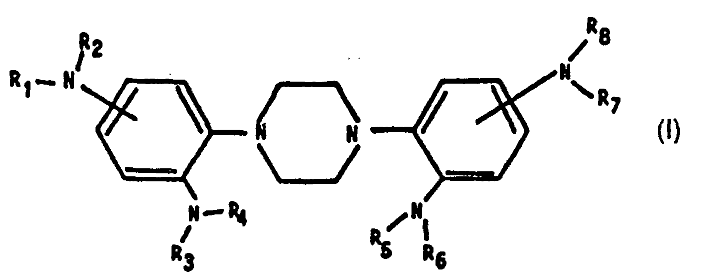 in which R₁ to R₈ independently of one another represent
- hydrogen,
- a (C₁₋₄)-alkyl group,
- a hydroxy-(C₂₋₃)-alkyl group,
- a (C₁₋₄)-alkoxy-(C₂₋₃)-alkyl group,
- an amino-(C₂₋₃)-alkyl group or
- a 2,3-dihydroxypropyl group,
with the proviso that the groups -NR₁R₂ and -NR₇R₈ are either in positions 4 and 4' or in positions 5 and 5',
and physiologically compatible salts thereof with inorganic and organic acids.

2. Piperazine derivatives corresponding to general formula (I) in claim 1, **characterized in that** the groups -NR₁R₂ and -NR₇R₈ and the groups-NR₃R₄ and -NR₅R₆ are identical.

3. An oxidation colorant for coloring keratin fibers, **characterized in that** a compound according to claim 1 or 2 is present as oxidation dye precursor.

4. An oxidation colorant as claimed in claim 3, **characterized in that** the compound corresponding to formula (I) is present in quantities of 0.001 to 10% by weight and, more particularly, 0.1 to 5% by weight, based on the colorant as a whole.

5. An oxidation colorant as claimed in claim 3 or 4, **characterized in that** it additionally contains an oxidation dye precursor of the secondary intermediate type.

6. An oxidation colorant as claimed in any of claims 3 to 5, **characterized in that** it additionally contains an oxidation dye precursor of the primary intermediate type.

7. An oxidation colorant as claimed in any of claims 3 to 6, **characterized in that** it additionally contains a substantive dye.

8. An oxidation colorant as claimed in any of claims 3 to 7, **characterized in that** it additionally contains a metal salt or a metal complex.

9. An oxidation colorant as claimed in claim 8, **characterized in that** the metal is selected from copper, manganese, cobalt, selenium, molybdenum, bismuth and ruthenium.

10. An oxidation colorant as claimed in claim 8 or 9, **characterized in that** the metal salt or the metal complex is present in quantities of 0.0001 to 1% by weight, based on the colorant as a whole.

11. The use of piperazine derivatives corresponding to general formula (I) in claim 1 for coloring keratin fibers.

## Revendications

1. Dérivés de pipérazine de formule générale (I), 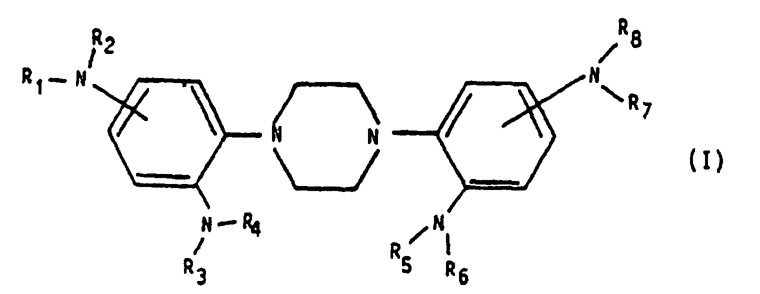 dans laquelle R₁ à R₈, indépendamment les uns des autres, représentent :
- de l'hydrogène,
- un groupe alkyle (en C₁-C₄),
- un groupe hydroxyalkyle (en C₂-C₃),
- un groupe (alkoxy en C₁-C₄) alkyle (en C₂-C₃),
- un groupe amino alkyle (en C₂-C₃), ou
- un groupe 2,3-dihydroxypropyle
avec la précision que les groupes -NR₁R₂ et -NR₇R₈ sont placés dans les positions 4 et 4' ou dans les positions 5 et 5',
ainsi que leurs sels physiologiquement compatibles avec des acides minéraux et organiques.

2. Dérivés de pipérazine de formule générale (I) selon la revendication 1,
**caractérisés en ce que**
les groupes -NR₁R₂ et -NR₇R₈ ainsi que -NR₃R₄ et -NR₅R₆ sont à chaque fois identiques.

3. Colorant par oxydation pour la teinture de fibres kératiniques,
**caractérisé en ce que**
comme produit précurseur de colorant par oxydation, un composé conformément à la revendication 1 ou la revendication 2, est contenu.

4. Colorant par oxydation conformément à la revendication 3,
**caractérisé en ce que**
le composé conformément à la formule (I) est contenu en quantités allant de 0,001 à 10 % en poids, en particulier de 0,1 à 5 % en poids, par rapport à la composition totale.

5. Colorant par oxydation selon la revendication 3 ou 4,
**caractérisé en ce qu'**
il contient en outre un produit précurseur de colorant par oxydation, du type agent de couplage.

6. Colorant par oxydation selon l'une des revendications 3 à 5,
**caractérisé en ce qu'**
il contient en outre un produit précurseur de colorant par oxydation, du type révélateur.

7. Colorant par oxydation selon l'une des revendications 3 à 6,
**caractérisé en ce qu'**
il contient en outre un colorant à action directe.

8. Colorant par oxydation selon l'une des revendications 3 à 7,
**caractérisé en ce qu'**
il contient en outre un sel métallique ou un complexe métallique.

9. Colorant par oxydation selon la revendication 8,
**caractérisé en ce que**
le métal est choisi parmi le cuivre, le manganèse, le cobalt, le sélénium, le molybdène, le bismuth et le ruthénium.

10. Colorant par oxydation selon la revendication 8 ou la revendication 9,
**caractérisé en ce que**
le sel métallique ou le complexe métallique est contenu en des quantités allant de 0,0001 à 1 % en poids par rapport à la totalité de la composition.

11. Utilisation de dérivés de la piperazine de formule (I) selon la revendication 1, pour la teinture des fibres kératiniques.
